# EUROPEAN PATENT APPLICATION

(11) **EP 2 740 493 A1**
(43) Date of publication of application: **11.06.2014**
(21) Application number: 12306521.1
(22) Date of filing: 05.12.2012
(51) Int. Cl.: A61K 47/48, A61P 35/00

(54) **Conjugates of the B-subunit of Shiga toxin for anticancer therapies**

(71) Applicant: Institut Curie, 75005 Paris (FR); Centre National de la Recherche Scientifique, 75794 Paris Cedex 16 (FR)
(72) Inventor: The designation of the inventor has not yet been filed
(74) Representative: Paris, Fabienne

(57) **Abstract**

Conjugates or pharmaceutical compositions comprising conjugates of the B-subunit of Shiga toxin that are useful for cancer therapies are disclosed. More specifically, the pharmaceutical compositions comprise STxB conjugated to at least one antimitotic agent such as auristatin or pharmaceutically acceptable salts thereof through a bifunctional linker. Processes to make the conjugates, as well as methods to treat cancers are also disclosed.

SEQUENCE LISTING

<110>  INSTITUT CURIE
       CENTRE NATIONAL DE LA RECHERCHE SCIENTIFIQUE

<120>  CONJUGATES OF THE B SUBUNIT OF SHIGA TOXIN FOR ANTICANCER
       THERAPIES

<130>  B10051-JAZ/SDU

<140>  EP XXXXXXXX.X
<141>  2012-12-05

<160>  1

<170>  PatentIn version 3.5

<210>  1
<211>  90
<212>  PRT
<213>  Artificial Sequence

<220>
<223>  B subunit of Shiga toxin

<400>  1

Met Lys Lys Thr Leu Leu Ile Ala Ala Ser Leu Ser Phe Phe Ser Ala
1               5                   10                  15

Ser Ala Leu Ala Thr Pro Asp Cys Val Thr Gly Lys Val Glu Tyr Thr
            20                  25                  30

Lys Tyr Asn Asp Asp Asp Thr Phe Thr Val Lys Val Gly Asp Lys Glu
        35                  40                  45

Leu Phe Thr Asn Arg Trp Asn Leu Gln Ser Leu Leu Leu Ser Ala Gln
    50                  55                  60

Ile Thr Gly Met Thr Val Thr Ile Lys Thr Asn Ala Cys His Asn Gly
65                  70                  75                  80

Gly Gly Phe Ser Glu Val Ile Phe Arg Cys
                85                  90

## Description

### Technical Field

The present invention relates to conjugates or pharmaceutical compositions comprising conjugates of the B-subunit of Shiga toxin that are useful for cancer therapies. More specifically, the pharmaceutical compositions comprise STxB conjugated to at least one antimitotic agent such as auristatin or pharmaceutically acceptable salts thereof through a bifunctional linker. Processes to make the conjugates, as well as methods to treat cancers are also disclosed.

### Background of the Invention

Antimitotic peptides and desipeptides are microtubule-targeted compounds that were originally isolated as natural products. The dolastatins, a class of antimitotic peptides, are natural cytotoxic pseudopeptides obtained as extracts from the marine organism *Dolabella auricularia.* Pettit et al (Biochem. Biophys. Acta 1971, 246, 225-232) characterized several toxic compounds in *Dolabella auricularia* that had cytotoxic activities against many cancerous cell lines. One product dolastatin 10 was discovered to be an agent that inhibited tubulin polymerization by binding to the vinca site on the tubulin like the anticancer drug vincristine (Pettit, G.R. Prog. Chem Org Nat. Prod, 1997, 701-79).

Analogs of dolastatin 10 have also been synthesized and were found to have biological activity. These analogs, known as auristatins are antimitotic short peptidic compounds that can be synthesized using combinatorial chemistry. Auristatin PE, auristatin E and auristatin F are linear peptides comprised of four amino acids, three of which are unique to this class of compounds. The structural differences between dolastatin 10 and auristatin resides in their C-terminal residue, in which the thiazolphenethyl amine of dolastatin is replaced by a norephedrine unit in auristatin E.

Antibody-drug conjugates, abbreviated as ADCs, are well known in the art for the clinical treatment of cancers. There are several already approved antibody-drug conjugates such as gemtuzumab ozogamicin (Mylotarg), and brentuximab vedotin (Adcetris) and others in clinical trials such as Trastuzumab emtansine (T-DM1), Inotuzumab ozogamicin (CMC-544), Glembatumumab vedotin (CDX-011, CR011-vcMMAE), lorvotuzumab mertansine (IMGN901), IMGN242(huC242-DM4), AN-152(AEZS-108), LMB2, Cantuzumab mertansine (huC242-DM1), AVE9633, SAR3419, CAT-8015, IMGN388, milatuzumab-doxorubicin, SGN-75 and AGS-16M8F. These agents display significant activities via antibody-dependent cellular cytotoxicity, complement dependent cytotoxicity and signal transduction. However, these activities are suboptimal even when combined with conventional cancer chemotherapy agents. Secondly the ADC's target only specific types of cancer.

Furthermore, the monoclonal antibodies in ADC's must attach to specific tumor cells and be released in the cancer cells. This is accomplished by using a cleavable linker in which the antibody is attached to the toxic payload. It is well known in the art that linkers play a critical role in conjugate potency and efficacy. Some linkers have short stabilities and half-lives. To be efficient, the chemotherapeutic drug should remain in stable linkage with the monoclonal antibody while in the circulation. After internalization the drug should be released once it is internalized into the target cells. If the linker is unstable drug toxicity may occur, as well as eventual masking of the tumor antigen with the mAb devoid of drug. See, Francisco et al Blood 2003:102:1458-1465.

Many ADC's use a linker such that when the conjugate enters the cell the antibody-drug conjugate is cleaved intracellularly using a cysteine protease such as cathepsin B. Thus, MMAE has been conjugated to a CD-30 specific monoclonal antibody, cAC10, and has been used to treat Hodgkin's lymphoma after failure of autologous stem cell transplants (See Younes et al The New England Journal of Medicine 363:19:1812-1821 (2010), Oakley et al, Clinical Cancer Research 16(3)888-897). In this respect, SGN-35 is an antibody conjugate that contains four molecules of monomethylauristatin E (MMAE). A cathepsin B-labile dipeptide was used as a linker to attach the MMAE to cAC10. Upon entry into the CD30-positive cells, the ADC is cleaved by cathepsin B. Thus, this system and conjugate is only adapted when the prodrug can encounter this particular enzyme and depends on the type of cancer and internal trafficking after endocytosis of the conjugate.

WO 02/088172 A2 describes pentapeptide compounds having cytotoxicity and prodrugs having targeting groups and pentapeptide moieties. Precursors having a reactive heterobifunctional linker are conjugated to an antibody used as a targeting moiety. This heterobifunctional linker on one end has a chemical moiety that reacts with a thiol group and on the other end has a hydrazine group.

Some of the problems encountered with ADC's were that there was inefficient internalization of the antibodies and linker instability.

The B-subunit of Shiga toxin (STxB) is nontoxic and exhibits high specificity for cancer cells expressing the cell surface glycosphingolipid receptor, globotriaosyl ceramide (Gb3). Once targeted to Gb3 cells, it undergoes cellular internalization and is transported in a retrograde fashion from the plasma membrane to the endoplasmic reticulum via early endosomes and the trans-Golgi network. This specific targeting avoids STxB recycling and degradation in the lysosomes. Furthermore, STxB could be detected in the tumor cells even five days after uptake. Falgieres et al Molecular Cancer Therapeutics 2008;7:2498-2508.

Gb3 expression has been shown in Burkitt's and centrofollicular lymphomas (Oosterwijk et al Int. J. Cancer 1991;48:848-54), in solid breast and solid ovarian tumors (LaCasse et al,Blood 1999; 94:2901-2010; Arab et al Oncl Res 1997;9:553-63) and in testicular seminomas (Ohyama et al Int, J, Cancer 1990;45:1040-4. Tumor cells of epithelial origin, as well as tumor-associated blood vessels and stroma are the origin of increased Gb3 expression in colorectal carcinoma (Falguieres et al Mol. Cancer Ther 2009;7:2498-508). In Maak et al Mol Cancer Ther 2011;10:1918-28, tumor-specific targeting of pancreatic cancer having Gb3 expression was demonstrated.

The B-subunit of Shiga toxin, a toxin vector, is known to be a universal carrier that can be used to target directly or indirectly the Gb3 receptor. U.S. Patent No. 6,613,882 describes a chimeric polypeptide of the formula B-X, where B is the B- subunit of Shiga toxin or functional equivalent thereof and X is a polypeptide of therapeutic significance. U.S. Patent 7,632,514 B2 describes a carrier having the formula STxB-Z (n)-Cys-X where STxB is the B-subunit of Shiga toxin Z is an amino acid linker without sulfhydryl groups and n is 0, 1, 2 or a polypeptide and Cys is Cysteine.

U.S. 2001/0152252 A1 describes the use of the B-subunit for Shiga toxin as a carrier for therapeutic agents, which is linked through a self-immolative linker, which is defined as a chemical moiety bond through two bonds to two molecules and which eliminates itself from the second molecule if the bond of the first molecule is cleaved.

Thus, there is a need in the art for targeting cancer drugs to cancer cells which is efficient, has less systemic side effects, achieves selective potency and whereby the drug is not downregulated. The linker must be stable in the bloodstream to limit the damage to the healthy tissue and release of the drug must be achieved in the cancer cell itself.

Although the B-subunit of Shiga toxin was known as a carrier it was unpredictable whether it would be effective when linked through a bifunctional linker to an antimitotic agent in such a manner that it would effectively protect a warm blooded animal from tumor growth and also induce tumor regression. Furthermore, the cytotoxicty of the conjugates or pharmaceutical compositions of the present invention is improved compared with other conjugates used to treat cancer.

### Summary of the Invention

The present invention provides a conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group.

In another aspect of the present invention comprises a conjugate comprising at least one B-subunit of Shiga toxin or a functional equivalent thereof covalently coupled to at least one antimitotic agent that has a reactive hydroxyl, amino or thiol group or pharmaceutically acceptable salts thereof through a linker, wherein said linker comprises a bifunctional linker connecting the at least one antimitotic agent that has a reactive hydroxyl, amino or thiol group or pharmaceutically acceptable salt thereof through a carbamate group and the at least one B-subunit of Shiga toxin or functional equivalent thereof through a disulfide group.

In another aspect the present invention comprises a conjugate comprising at least one B-subunit of Shiga toxin or a functional equivalent thereof covalently coupled to at least one antimitotic agent that has a reactive hydroxyl group or pharmaceutically acceptable salts thereof through a linker, wherein said linker comprises a bifunctional linker connecting the at least one antimitotic agent that has a reactive hydroxyl group or pharmaceutically acceptable salt thereof through a carbamate group and the at least one B-subunit of Shiga toxin or functional equivalent thereof through a disulfide group.

In another embodiment the present invention comprises a conjugate comprising at least one B-subunit of Shiga toxin or a functional equivalent thereof covalently coupled to at least one antimitotic agent that has a reactive hydroxyl group selected from the group comprising halichomdrin B, maytansinoids, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, platinum derivatives, platinum drugs including cisplatinum, carboplatin, oxiplatin, platinum (IV), auristatin, auristatin E and monomethyl auristatin E, pharmaceutically acceptable salts thereof and mixtures thereof through a linker, wherein said linker comprises a bifunctional linker connecting the at least one antimitotic agent that or pharmaceutically acceptable salt thereof through a carbamate group and the at least one B-subunit of Shiga toxin or functional equivalent thereof through a disulfide group.

In another aspect the present invention comprises a conjugate comprising at least one B-subunit of Shiga toxin or a functional equivalent thereof covalently coupled to at least one auristatin or pharmaceutically acceptable salts thereof through a linker, wherein said linker comprises a bifunctional linker connecting the at least one auristatin or pharmaceutically acceptable salt thereof through a carbamate group and the at least one B-subunit of Shiga toxin or functional equivalent thereof through a disulfide group.

The conjugate can contain auristatin E or monomethyl auristatin E as the antimitotic agent.

In another aspect the conjugate comprises the functional equivalent of the Shiga B toxin, which is selected from the group of Shiga-like toxin 1, Shiga-like toxin 2 , Shiga-like toxin 2c, Shiga-like toxin 2d1, Shiga-like toxin 2d2, Shiga-like toxin 2e, Shiga-like toxin 2f and Shiga-like toxin 2y,verotoxin-1, verotoxin-2, verotoxin 2c or verotoxin 2v.

In yet another aspect the conjugate comprises a carbamate group that is coupled to the at least one antimitotic agent and a disulfide pyridine group that is coupled to the at least one B-subunit of Shiga toxin or functional equivalents thereof through the free thiol group.

In yet another aspect the conjugate comprises a carbamoyl chloride group that is coupled to the at least one antimitotic agent and a di-thiol pyridine group that is coupled to the at least one B-subunit of Shiga toxin or functional equivalents thereof through the free thiol group.

In still another aspect the carbamoyl chloride group has the formula NCOCI and the di-thiol group is a di-thiol pyridine having the formula S₂-C₅H₅N in said linker.

A pharmaceutical composition comprising a conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group and a pharmaceutically acceptable vehicle is another aspect of the present invention.

In another embodiment a pharmaceutical composition comprising a conjugate comprising at least one B-subunit of Shiga toxin or a functional equivalent thereof covalently coupled to at least one antimitotic agent that has a reactive hydroxyl, amino or thiol group or pharmaceutically acceptable salts thereof through a linker, wherein said linker comprises a bifunctional linker connecting the at least one antimitotic agent that has a reactive hydroxyl, amino or thiol group or pharmaceutically acceptable salt thereof through a carbamate group and the at least one B-subunit of Shiga toxin or functional equivalent thereof through a disulfide group and a pharmaceutically acceptable vehicle is disclosed.

In another aspect the pharmaceutical composition of the present invention comprises a conjugate comprising at least one B-subunit of Shiga toxin or a functional equivalent thereof covalently coupled to at least one antimitotic agent that has a reactive hydroxyl group or pharmaceutically acceptable salts thereof through a linker, wherein said linker comprises a bifunctional linker connecting the at least one antimitotic agent that has a reactive hydroxyl group or pharmaceutically acceptable salt thereof through a carbamate group and the at least one B-subunit of Shiga toxin or functional equivalent thereof through a disulfide group and a pharmaceutically acceptable vehicle.

In another embodiment the pharmaceutical composition of the present invention comprises a conjugate comprising at least one B-subunit of Shiga toxin or a functional equivalent thereof covalently coupled to at least one antimitotic agent that has a reactive hydroxyl group selected from the group comprising halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, platinum derivatives, platinum drugs including cisplatinum, carboplatin, oxiplatin, platinum (IV)auristatin, auristatin E and monomethyl auristatin E, pharmaceutically acceptable salts thereof and mixtures thereof through a linker, wherein said linker comprises a bifunctional linker connecting the at least one antimitotic agent that or pharmaceutically acceptable salt thereof through a carbamate group and the at least one B-subunit of Shiga toxin or functional equivalent thereof through a disulfide group and a pharmaceutically acceptable vehicle.

In yet another aspect the pharmaceutical composition of the present invention comprises a conjugate comprising at least one B-subunit of Shiga toxin or a functional equivalent thereof covalently coupled to at least one auristatin or pharmaceutically acceptable salts thereof through a linker, wherein said linker comprises a bifunctional linker connecting the at least one auristatin or pharmaceutically acceptable salt thereof through a carbamate group and the at least one B-subunit of Shiga toxin or functional equivalent thereof through a disulfide group and a pharmaceutically acceptable vehicle.

The conjugate can contain auristatin E or monomethyl auristatin E.

The pharmaceutical compositions can also comprise additional therapeutic agents. Examples of the additional therapeutic agent include an analgesic, an antimicrobial, an antibacterial, an antiviral, an antibiotic, an anti-inflammatory, an antioxidant, an antihistamine, an antipruritic, an antipyretic, an additional anti-cancer agent and combinations thereof.

In another embodiment, the pharmaceutical compositions comprise at least 5 auristatin molecules that are coupled to the B-subunit of Shiga toxin.

A pharmaceutical composition comprising a first conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group and/or a second conjugate comprising at least one B- subunit of Shiga toxin or a functional equivalent thereof covalently coupled to at least one cancer antigen through a linker, wherein said linker comprises a bifunctional linker connecting the at least one cancer antigen through a carbamate group and the at least one B-subunit of Shiga toxin or functional equivalent thereof through a disulfide group and a pharmaceutically acceptable vehicle forms another aspect of the invention.

The first conjugate, described above, can be synthesized separately from the second conjugate and the pharmaceutical compositions can be mixed together and administered simultaneously. Alternatively after separate synthesis of the two conjugates, the first conjugate can be administered first and the second conjugate administered after the first conjugate or the second conjugate can be administered first and the first conjugate administered after the second conjugate.

Adjuvants can also be administered with the second conjugate.

A method of treating cancer said method comprising administering to a warm blooded animal in need of such treatment a pharmaceutical composition comprising a conjugate comprising at least one B-subunit of Shiga toxin or a functional equivalent thereof covalently coupled to at least one antimitotic agent, as described herein, or pharmaceutically acceptable salts thereof through a linker, wherein said linker comprises a bifunctional linker connecting the at least one antimitotic agent, as described herein, or pharmaceutically acceptable salt thereof through a carbamate group and the at least one B-subunit of Shiga toxin or functional equivalent thereof through a disulfide group and a pharmaceutically acceptable vehicle is yet another aspect of the present invention.

The pharmaceutical composition as described herein can be administered topically, enterally, parentally, intravenously or orally and can treat cancers such as acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ,* embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ*, lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vaginal cancer, and vulvar cancer.

A method of targeting the conjugate, as described herein, or the pharmaceutical composition, as described herein, comprising at least one antimitotic agent, as described herein, or pharmaceutically acceptable salts thereof to Gb3 containing cancer cells said method comprising administering the conjugate, as described herein or the pharmaceutical composition, as described herein, wherein said targeting involves retrograde transport through the cancer cell of said conjugate or pharmaceutical composition and avoidance of translocation into the lysosomes.

The conjugate, as described herein, or the pharmaceutical composition, as described herein, for use as a medicament, preferable in treating cancer is yet another aspect of the invention.

Use of the conjugates as described herein or the pharmaceutical compositions as described herein for the manufacture of a medicament preferably to treat cancer is yet another aspect of the invention.

A method of making a conjugate, as described herein, said method comprising synthesizing a bifunctional linker, reacting a Boc protected antimitotic agent, such as auristatin E, with said bifunctional linker, deprotecting the Boc group on said antimitotic agent to form a coupled bifunctional linker- antimitotic agent and coupling the bifunctional linker-antimitotic agent to the B-subunit of Shiga toxin is a further aspect of the invention.

Alternatively, the conjugate can be made by reacting a hydroxyl group of a Boc protected antimitotic agent, as described herein, to form an activated Boc protected antimitotic agent and coupling said activated Boc protected antimitotic agent with a bifunctional linker, deprotecting the Boc group to form a coupled bifunctional linker- antimitotic agent and coupling the bifunctional linker- antimitotic agent to the B-subunit of Shiga toxin is yet a further aspect of the invention.

Other aspects and embodiments are set forth below, or will readily arise from the following description of the preferred embodiments.

### Brief Description of the Figures

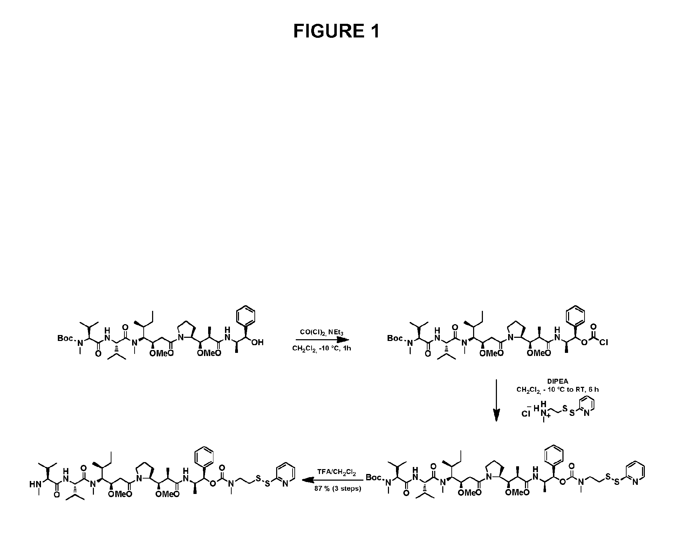

Fig. 1 is a schematic representation of a synthesis of monomethyl auristatin (MMAE) conjugate with a linker in the presence of 4-diisopropylethyl amine (DIPEA) using Boc chemistry to protect the terminal amine group. The molecule is then deprotected.

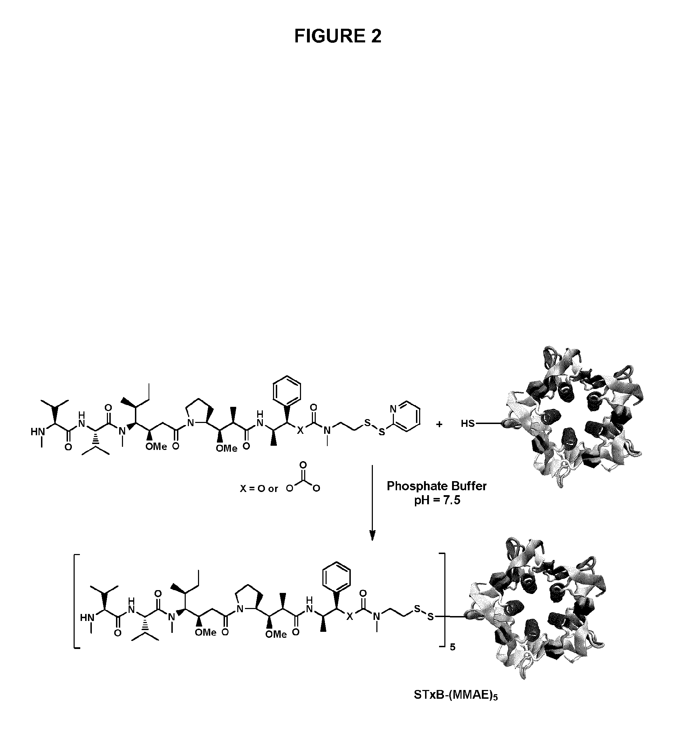

Fig. 2 is a schematic representation of the coupling of STxB to the monomethyl auristatin linker. The coupling is performed in a mixture of DMSO and water containing buffered phosphate at pH 7.5. The ligation with the STxB and the monomethyl auristatin(MMAE) moiety was done overnight at room temperature.
Fig. 3 are photographs showing the internalization of the STxB-(MMAE)₅ in HT-29 cells. This test was performed with the aid of antibodies that are specific for the Golgi apparatus and present their localization therein. The superposition to the right of the two photographs validates that retrograde transport occurs even in the presence of the cytotoxic agent.
Fig. 4 is a graph showing a cytotoxic test using auristatin alone and with the conjugate STxB-(MMAE)₅ in HT29 cells with or without D, L-treo-1-phenyl-2-palmitoylamino-3-morpholino-1-propanol (PPMP) an inhibitor of glucosylceramide synthesis.

### Description of the Preferred Embodiments

By "retrograde transport" is meant that the B-subunit enters the cell after binding to the Gb3 receptor through a clathrin-independent endocytosis, reaches the interface between the early endosome and the trans-Golgi network and is transported to the Golgi via the retrograde pathway and is transported from the Golgi to the endoplasmic reticulum. The retrograde pathway is distinct from the well known pathway used by the mannose-6-phosphate receptor.

The abbreviation "Gb3" receptor means the globotriasylceramide receptor.

The term "immunogenic" means capable of inducing an immune response.

The term "adjuvant" as used herein means any substance that helps or enhances the pharmacological effect of a drug or increases the ability of an antigen to stimulate the immune system.

The term "warm blooded animals" as used herein includes birds and mammals.

Examples of birds that can be treated with the compositions and methods of the invention include blue birds, cardinals, doves, eagles, geese, turkeys, chickens, hens, ducks, quails, herons, sparrows, woodpeckers, owls, parrots and the like.

The term "mammal" encompasses any of various warm-blooded vertebrate animals of the class Mammalia, including humans, characterized by a covering of hair on the skin and, in the female, milk-producing mammary glands for nourishing the young. The present invention is not limited to treating humans, but also encompasses veterinary applications, especially since it is well known that animals also can have different medical pathologies.

Examples of mammals that can be treated with the compositions and methods of the present invention include humans, domestic animals such as dogs and cats, horses, mice, goats, deer, cows, rabbits, zoo animals, bears, monkeys, apes, elks, bison, although this invention may be applied to other mammalian species as well.

"Dendritic cells" as used herein means any type of dendritic cells including plasmacytoid dendritic cells, CD8⁺ dendritic cells, CD8- dendritic cells, myeloid dendritic cells, inflammatory dendritic cells, Tip dendritic cells and Langerhans cells. Thus, dendritic cells include migratory dendritic cells such as Langerhans cells and dermal dendritic cells, lymphoid tissue resident dendritic cells such as thymic and splenic dendritic cells and inflammatory dendritic cells.

By "functional equivalent thereof" with respect to the B-subunit of Shiga toxin in the conjugates, first conjugates and pharmaceutical compositions, described herein, is meant that the toxin can bind to Gb3 cells and/or they can internalize the at least one antimitotic agent or pharmaceutically acceptable salts thereof.

By "functional equivalent thereof" with respect to the B-subunit of Shiga toxin with respect to the second conjugate containing the cancer antigen in the pharmaceutical composition, described herein, is meant that the toxin acts immunogenically like the B-subunit of Shiga toxin and can bind to Gb3 cells and/or they can internalize the at least one cancer antigen such that the at least one antigen can be presented to the MHC class I pathway or the MHC class I and class II pathways on the same antigen presenting cells. Furthermore, the functional equivalents can target dendritic cells.

"Treating," as used herein means giving medical care or attention to a warm blooded animal and/or the combating of a disease especially via targeted anti-cancer therapy.

"Antimitotic agents" as used herein means an agent that inhibits cancer growth by stopping cell division. For example, dolastatins are agents that inhibit microtubule polymerization thereby arresting cell division.

By "at least one antimitotic agent" as used herein means that a sole antimitotic agent, as described herein, can be used in the conjugate, described herein, or pharmaceutical compositions, described herein, or more than one antimitotic agent can be used in the conjugate, as described herein, or pharmaceutical compositions, as described herein, such as 2, 3, 4 or 5 antimitotic agents.

The terminology "at least one auristatin" includes herein auristatin E, monomethyl auristatin E and mixtures thereof.

The term "carbamate group" when referring to the linker means a NCOCI group or a NCOO group.

By "consisting essentially of" means that the major elements are present in the conjugate or pharmaceutical compositions, but also minor ingredients that do not materially affect the conjugate or pharmaceutical compositions can also be present.

The terms, comprising, consisting of and consisting essentially of can be interchanged throughout the specification.

By the term "conjugate" is meant at least two entities that are joined together, linked together or coupled together.

A "bifunctional linker" means a linker that has two chemically reactive functional groups.

A "heterobifunctional linker" as described herein means a linker that has two different chemically reactive functional groups.

More specifically, the invention comprises a conjugate comprising at least one B-subunit of Shiga toxin or a functional equivalent thereof, which is covalently coupled to at least one antimitotic agent or pharmaceutically acceptable salts of the antimitotic agent through a linker. The B-subunit of Shiga toxin acts as a carrier for the at least one antimitotic agent or pharmaceutically acceptable salts thereof and targets cancer cells. By targeting cancer cells and more specifically the Gb3 receptor found on cancer cells and in the vasculature of cancer cells, the antimitotic cancer agent has sufficient cellular uptake, low cytotoxicity and especially low cytotoxicity for the healthy non-cancerous cells and few systemic and off-target effects. Furthermore, Gb3 is highly expressed in tumors with more than 10⁷ binding sites per cell.

Thus, the conjugates and the pharmaceutical compositions, described herein, are useful to effectively protect a warm blooded animal from tumor growth and also induce tumor regression. Furthermore, the cytotoxicty of the conjugates or pharmaceutical compositions of the present invention is greatly improved compared with other conjugates used to treat cancer.

The sequence of the B-subunit of Shiga toxin has been described in Strockbine et al., J. Bacteriol, 170, 1116-22 (1988). The functional equivalent of the B-subunit of Shoiga toxin is one in which the toxin can bind to the Gb3 receptor. In the case of cancer antigens, the functional equivalent of the B-subunit of Shiga toxin causes the internalization of these antigens and their presentation to the MHC class I pathway or both MHC class I and class II pathways. Thus, the B-subunits of Shiga-like toxins from *E. coli* such as Shiga-like toxin 1, Shiga-like toxin 2 and the Shiga-like toxin 2 variants such as Shiga-like toxin 2c, Shiga-like toxin 2d1, Shiga-like toxin 2d2, Shiga-like toxin 2e, Shiga-like toxin 2f and Shiga-like toxin 2y can all be considered functional equivalents. In an embodiment the B-subunits of verotoxin-1 or verotoxin-2 or verotoxin 2c or verotoxin 2v from *E. coli* can be used instead of the B-subunit of Shiga toxin in the formulation of the conjugate or pharmaceutical compositions of the present invention. It should be noted that verotoxin-1 is an alternative name for Shiga-like toxin 1 and that verotoxin-2 is an alternative name for Shiga-like toxin 2.

The B-subunit of Shiga toxin or the functional equivalent thereof is devoid of toxin activity and hence is not toxic to mammals. Lacking toxicity does not affect the binding of the B-subunit of Shiga toxin or functional equivalents thereof to Gb3 receptors or the entry into the MHC class I or MHC class I or II pathways with respect to the cancer antigens.

The binding to the Gb3 receptor may be evaluated by methods known in the art such as those described by Tarrago-Trani in Protein Extraction and Purification, 39:pp 170-176 (2004) or Nishikawa et al in Chem Pharm Bull April 54(4): pp.522-7 (2006), which are incorporated herein by reference.

In one embodiment the B-subunit of Shiga toxin has the sequence: COOH-MKKTLLIAASLSFFSASALATPDCVTGKVEYTKYNDDDTFTVKVGDK ELFTNRWNLQSLLLSAQITGMTVTIKTNACHNGGGFSEVIFRC-NH2 (SEQ ID NO: 1).

The at least one antimitotic agents or pharmaceutically acceptable salts of the antimitotic agents that can be used in the conjugates, described herein, or pharmaceutical compositions, described herein, include allocolcicine, halichomdrin B, colchicines, colchicine derivatives, dolastatin, dolastatin derivatives, maytansinoids, rhizoxin, taxol, taxol derivatives, thicolchicine, tritylcysteine, vinblastine sulfate, vincristine sulfate, platinum derivatives, platinum drugs including cisplatinum, carboplatin, oxiplatin, platinum (IV), auristatin F, monomethyl auristatin F, auristatin E, monomethyl auristatin E, symplostatins, dolastatin 3, dolastatin 10, dolastatin 15, dolastatin 16, dolastatin 17, dolastatin 18, cemadotin, TZT-1027, tasidotin, soblidotin, HTI-286 and mixtures thereof.

In another aspect the at least one antimitotic agent or pharmaceutically acceptable salts of the antimitotic agents that can be used in the conjugates, described herein, or pharmaceutical compositions, described herein, have a secondary amine at the amine terminal of the antimitotic agent or an hydroxyl or an amino or thiol group in its structure.

In another embodiment, the at least one antimitotic agent or pharmaceutically acceptable salts of the antimitotic agents that can be used in the conjugates, described herein, or pharmaceutical compositions, described herein, have a reactive hydroxyl group for coupling to the bifunctional linker. Examples of antimitotic agents that have a reactive hydroxy group include halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins and derivatives, doxorubicin and derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide platinum derivatives, platinum drugs including cisplatinum, carboplatin, oxiplatin, platinum (IV) and mixtures thereof.

Examples of dolastatin derivatives which include a hydroxyl group are auristatin, auristatin E, monomethyl auristatin E and mixtures thereof and the like.

The at least one antimitotic agent,as described herein can modified in such a manner to insert a reactive hydroxyl, amino or thiol group.

The pharmaceutically acceptable salts of the antimitotic agents that are used in the conjugates, described herein, and the pharmaceutical compositions, described herein, include those that are organic or inorganic salts of the antimitotic agents. These are well known and described in the Physician's Desk Reference, The Merck Index and Goodman and Gilman's The Pharmacological Basis of Therapeutics. The pharmaceutically acceptable salts are, for example, sodium, potassium, ammonium, calcium and magnesium and salts formed with inorganic acids such as hydrochloric acid, hydrobromic acid, phosphoric acid, sulfuric acid and the like or salts formed with organic acids such as oxalic acid, fumaric acid, tartaric acid, malonic acid, acetic acid, citric acid, benzoic acid and the like.

The conjugate or pharmaceutical compositions, all as described herein, can have at least one antimitotic agent coupled to the B-subunit of Shiga toxin. It can have 1 to 3 antimitotic agents coupled to the B-subunit of Shiga toxin or it can have 1 to 5 antimitotic agents coupled to the B-subunit of Shiga toxin. For example, at least one auristatin molecule can be coupled to the B-subunit of Shiga toxin or 1 or 2 auristatins to the B-subunit of Shiga toxin or 1 to 3 auristatins to the B-subunit of Shiga toxin or 1 to 4 auristatins to the B-subunit of Shiga toxin, or 1 to 5 auristatins can also be coupled to the B-subunit of Shiga toxin. To have the most cytotoxicity generally five auristatin molecules are coupled to the B-subunit of Shiga toxin.

The B-subunit of Shiga toxin is coupled to the antimitotic agent through a bifunctional linker. This bifunctional linker has two chemical reactive groups that can be used to couple the B-subunit of Shiga toxin to the antimitotic agent through covalent bonds. This bifunctional linker can be a heterobifunctional linker having two different chemical reactive groups.

In one embodiment this bifunctional linker has a chemically reactive carbamate group and a disulfide group. The carbamate group couples the at least one antimitotic agent and the disulfide group couples the at least one B-subunit of Shiga toxin or functional equivalents thereof through the free thiol group.

In yet another embodiment this bifunctional linker has a chemically reactive carbamoyl chloride group and a di-thiol pyridine group. The carbamoyl chloride group couples the at least one antimitotic agent and the di-thiol pyridine group couples the at least one B-subunit of Shiga toxin or functional equivalents thereof through the free thiol group.

In another aspect the carbamoyl chloride group has the formula NCOCI and the di-thiol group is a di-thiol pyridine group having the formula S₂-C₅H₅N.

Linkers such as 3-maleimidobenzoic acid N-hydroxyysuccinimide ester (MBS), N-succinimidyl 3-[2-pyridyldithio]-propionate (SPDP), [S-(N-succinimidyl)thioacetate] (SATA), [(N-succinimidiyloxy carbonyl)1-methyl-1-(2-pyridyldithio) toluene] (SMPT), 2-iminothiolane (2-IT), (Sulfosuccinimidyl N-[3-(Acetylthio)-3-methylbutyryl)-beta-alanine]) (sulfoNHS-ATMBA), thioimidates such as AMPT and M-CDPT and 2-[N-chlorocarbonyl)-N-methylamino]1-ethyl 2-pyridyldisulfide might be used. The chemical structures of these linkers are set forth below:

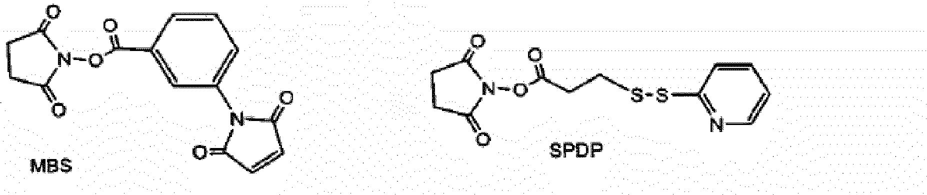

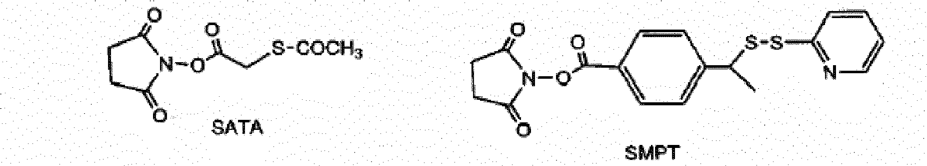

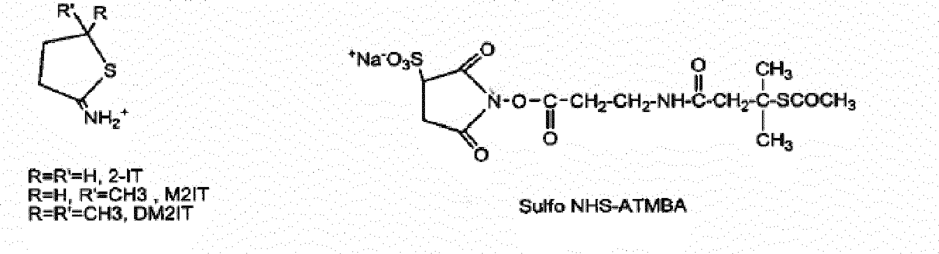

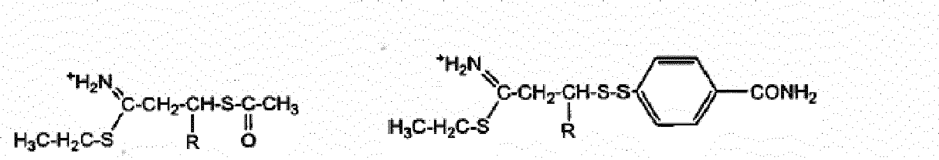

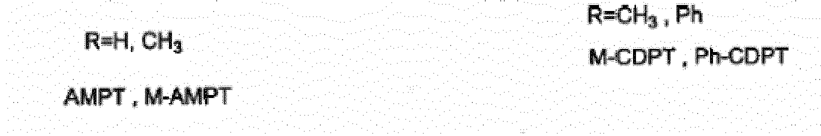

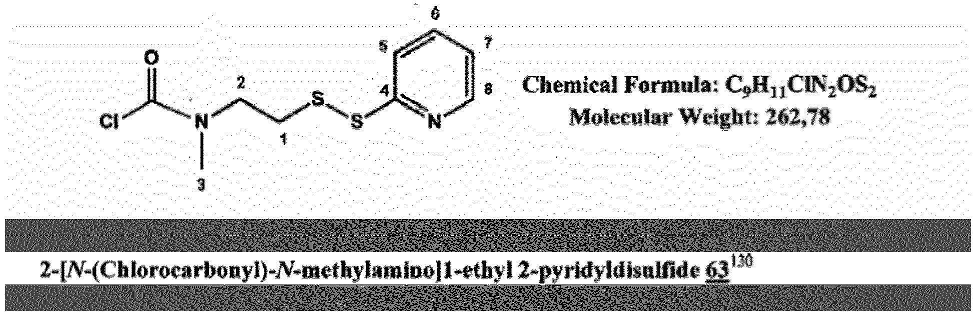

The bifunctional linker is coupled to the B-subunit of Shiga toxin and to the antimitotic agent or pharmaceutically acceptable salt thereof or auristatin or the pharmaceutically acceptable salt thereof by the method described herein. The above linkers can be cleaved by the reductive cleavage of the disulfide bond. This cleavage occurs in the cell in the presence of glutathione.

In another aspect the bifunctional linker is a self-immolative linker comprising a disulfide bond and an aromatic spacer moiety that participates in the destruction after cleavage with the disulfide bond.

In yet another embodiment a pharmaceutical composition comprising the conjugate, as described herein, and a pharmaceutically acceptable vehicle is encompassed by the present invention. More specifically, this pharmaceutical composition comprises a conjugate comprising at least one B-subunit of Shiga toxin or a functional equivalent thereof, which is covalently coupled to at least one antimitotic agent, as described herein, or pharmaceutically acceptable salts of the antimitotic agent through a bifunctional linker, as described herein.

In another aspect the pharmaceutical composition of the present invention comprises a conjugate comprising at least one B-subunit of Shiga toxin or a functional equivalent thereof covalently coupled to at least one antimitotic agent that has a reactive hydroxyl, amino or thiol group or pharmaceutically acceptable salts thereof through a linker, wherein said linker comprises a bifunctional linker connecting the at least one antimitotic agent that has a reactive hydroxyl, amino or thiol group or pharmaceutically acceptable salt thereof through a carbamate group and the at least one B-subunit of Shiga toxin or functional equivalent thereof through a disulfide group and a pharmaceutically acceptable vehicle.

In another aspect the pharmaceutical composition of the present invention comprises a conjugate comprising at least one B-subunit of Shiga toxin or a functional equivalent thereof covalently coupled to at least one antimitotic agent that has a reactive hydroxyl group or pharmaceutically acceptable salts thereof through a linker, wherein said linker comprises a bifunctional linker connecting the at least one antimitotic agent that has a reactive hydroxyl group or pharmaceutically acceptable salt thereof through a carbamate group and the at least one B-subunit of Shiga toxin or functional equivalent thereof through a disulfide group and a pharmaceutically acceptable vehicle.

In another embodiment the pharmaceutical composition of the present invention comprises a conjugate comprising at least one B-subunit of Shiga toxin or a functional equivalent thereof covalently coupled to at least one antimitotic agent that has a reactive hydroxyl group selected from the group comprising halichomdrin B, maytansinoids, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, platinum derivatives, platinum drugs including cisplatinum, carboplatin, oxiplatin, platinum (IV), auristatin, auristatin E and monomethyl auristatin E, pharmaceutically acceptable salts thereof and mixtures thereof through a linker, wherein said linker comprises a bifunctional linker connecting the at least one antimitotic agent or pharmaceutically acceptable salts thereof through a carbamate group and the at least one B-subunit of Shiga toxin or functional equivalent thereof through a disulfide group and a pharmaceutically acceptable vehicle.

In another aspect the pharmaceutical composition of the present invention comprises a conjugate comprising at least one B-subunit of Shiga toxin or a functional equivalent thereof covalently coupled to at least one auristatin or pharmaceutically acceptable salts thereof through a linker, wherein said linker comprises a bifunctional linker connecting the at least one auristatin or pharmaceutically acceptable salt thereof through a carbamate group and the at least one B-subunit of Shiga toxin or functional equivalent thereof through a disulfide group and a pharmaceutically acceptable vehicle.

The conjugate in the pharmaceutical composition can contain auristatin E or monomethyl auristatin E.

A pharmaceutical composition comprising a first conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group and/or a second conjugate comprising at least one B- subunit of Shiga toxin or a functional equivalent thereof covalently coupled to at least one cancer antigen through a linker, wherein said linker comprises a bifunctional linker connecting the at least one cancer antigen through a carbamate group and the at least one B-subunit of Shiga toxin or functional equivalent thereof through a disulfide group and a pharmaceutically acceptable vehicle forms another aspect of the invention.

The first conjugate, described above, can be synthesized separately from the second conjugate and the pharmaceutical composition can be mixed together and administered simultaneously. Alternatively after separate synthesis of the two conjugates, the first conjugate can be administered first and the second conjugate administered after the first conjugate or the second conjugate can be administered first and the first conjugate administered after the second conjugate.

The cancer antigens can be any cancer antigens that treat the specific cancers described herein. For example, cancer cell antigens can include WT1,MUC1, LMP2, HPV E6, E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinase3, bcr-abl, Tyrosinase, Survivin, PSA, hTERT, Sarcoma translocation breakpoints, EphA2, PAP, ML-IAP, AFP, EpCAM, ERG, NA17, PAX3, ALK, androgen receptor, Cyclin B1, Polysialic acid, MYCN, RhoC, TRP-2, GD3, Fucosyl GM1, Mesothelin, PSCA, MAGE A1, sLe, CYP1b1, PLAC1, GM3m, BORIS, Tn, MAGE-B2, DAM-10, SAGE, RAGE and the like.

The pharmaceutically acceptable vehicle can be any acceptable carrier, adjuvant or vehicle that does not interfere with the pharmaceutical activity of the pharmaceutical composition and is not toxic to the host to which the pharmaceutical composition is administered. It includes solvents, dispersion media, coatings, absorption delaying agents and the like. These pharmaceutically acceptable vehicles are described in Remington's Pharmaceutical Sciences 21st edition 2005. An acceptable carrier can be, for example, saline, buffered saline and the like. It can be added to the pharmaceutical composition after its formulation.

The pharmaceutical compositions, as described herein, can also contain an additional therapeutic agent. This additional therapeutic agent can either be coupled to the conjugate through the B-subunit or administered simultaneously with the conjugate described herein or pharmaceutical compositions as described herein. It can also be administered before or after the pharmaceutical compositions described herein. This additional therapeutic agent is selected from the group of an analgesic, an antimicrobial, an antibacterial, an antiviral, an antibiotic, an anti-inflammatory, an antioxidant, an antihistamine, an antipruritic, an antipyretic, an additional anti-cancer agent and combinations thereof.

Adjuvants can also be administered with the pharmaceutical compositions that contain the second conjugate that has cancer antigens. Examples of adjuvants include alum, GM-CSF and the CpG oligodeoxynucleotides or a CpG-like oligodeoxynucleotides, Freund's incomplete adjuvant (IFA)m MF59®, poly(I:C), monophosphoryl lipid A (MPLA), TLR5 ligands such as bacterial flagellin, TLR7/8 ligands such as imiquimod, gardiquimod and R848, NOD2 ligands such as muramyl dipeptides and mixtures thereof.

A method of treating cancer by administering to a warm blooded animal in need of such treatment pharmaceutical compositions, as described herein, comprising a conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker and a pharmaceutically acceptable vehicle is another aspect of the invention. The bifunctional linker can be a heterobifunctional linker and have a carbamate group and a disulfide group.

Yet another embodiment of the invention is a method of treating cancer by administering to a warm blooded animal in need of such treatment a pharmaceutical composition, as described herein, comprising a conjugate comprising at least one B- subunit of Shiga toxin or a functional equivalent thereof covalently coupled to at least one auristatin or pharmaceutically acceptable salts of auristatin through a linker, wherein said linker comprises a bifunctional linker having a carbamoyl chloride group and a di-thiol pyridine group and a pharmaceutically acceptable vehicle.

The pharmaceutical compositions, as described herein, can be administered topically, enterally, parentally, intravenously or orally. The pharmaceutical compositions, described herein, can also be administered mucosally or systemically. If delivered systemically it is generally through transdermal, subcutaneous or intramuscular routes. In one embodiment the pharmaceutical compositions of the present invention and the adjuvants, if cancer antigens are present in the pharmaceutical composition, are administered intramuscularly. In another embodiment the pharmaceutical compositions of the present invention are injected directly into the tumor.

The pharmaceutical compositions, as described herein, can be administered alone or additional chemotherapy can be used either before or after the administration of any one these pharmaceutical compositions.

The cancer that can be treated by administering the pharmaceutical compositions as described herein are acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt Lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ*, embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ*, lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vulvar cancer and vaginal cancer.

A method of targeting the conjugate, as described herein, or the pharmaceutical compositions, as described herein, comprising at least one antimitotic agent or pharmaceutically acceptable salts thereof to Gb3 containing cancer cells said method comprising administering the conjugate, as described herein or the pharmaceutical composition, as described herein, wherein said targeting involves retrograde transport through the cancer cell of said conjugate or pharmaceutical composition and avoidance of translocation into the lysosomes is yet another aspect of the invention. This administration can be *in vivo* to a warm blooded animal in need of such treatment.

The retrograde transport of the pharmaceutical compositions, as described herein, or the conjugate, as described herein, is an important feature of the present invention since unlike transportation of antimitotic agents with monoclonal antibodies there is avoidance of the translocation into lysosomes, which can degrade the antimitotic agent. Furthermore, since tumor blood flow is not always optimal and high interstitial pressure within the tumor can prevent the monoclonal antibodies from binding. Thus, the present conjugate, as described herein, or pharmaceutical compositions, as described herein, has advantages over those antimitotic agents delivered to cancer cells via antibodies since they can protect the warm blooded animal from tumor growth and induce tumor regression.

The conjugate, as described herein, or the pharmaceutical compositions, as described herein, for use as a medicament, preferably in treating cancer is yet another aspect of the invention

Also use of the conjugate, as described herein, or the pharmaceutical compositions, as described herein, for the manufacture of a medicament to treat cancer is encompassed by the present invention.

The amount of the at least one antimitotic agent or pharmaceutically acceptable salt thereof used in the pharmaceutical compositions, as described herein, and/or the methods, as described herein, depends on the mode of administration and can vary with each different formulation. The amount is generally calculated based on the body weight of the warm blooded animal to which it is administered. The dosage administered also depends on the type of cancer, the severity of the cancer and whether the warm blooded animal has other diseases or medical conditions.

Usually the conjugate present in the pharmaceutical compositions, as described herein, or in any one of the methods, as described herein, will contain between 2 to 40 mg of conjugate/kilogram, which contains between .073 to 1.5 of the at least one antimitotic agent or pharmaceutically acceptable salt thereof, as described herein. In another aspect the conjugate, as described herein will contain between 0.1 to 50 mg of conjugate/kilogram, which contains between .004 to 1.8 of the at least one antimitotic agent or pharmaceutically acceptable salt thereof. In yet another aspect the conjugate, as described herein, will contain between 8 to 25 mg of conjugate/kilogram, which contains between .30 to 0.92 of the at least one antimitotic agent or pharmaceutically acceptable salt thereof, as described herein.

In another aspect the conjugate present in the pharmaceutical compositions, as described herein, or in any one of the methods, as described herein, will contain between 2 to 40 mg of conjugate/kilogram, which contains between .073 to 1.5 of the at least one auristatin or pharmaceutically acceptable salt thereof. In another aspect the conjugate, as described herein will contain between 0.1 to 50 mg of conjugate/kilogram, which contains between .004 to 1.8 of the at least one auristatin or pharmaceutically acceptable salt thereof. In yet another aspect the conjugate, as described herein, will contain between 8 to 25 mg of conjugate/kilogram, which contains between .30 to 0.92 of the at least one auristatin or pharmaceutically acceptable salt thereof.

A method of making a conjugate as described herein said method comprising synthesizing a bifunctional linker, reacting a Boc protected antimitotic agent, as described herein, with said bifunctional linker, deprotecting the Boc group on said antimitotic agent, as described herein, to form a coupled bifunctional linker- antimitotic agent and coupling the bifunctional linker- antimitotic agent to the B- subunit of Shiga toxin.

Alternatively the conjugate can be made by reacting a hydroxyl group of a Boc protected antimitotic agent, as described herein, to form an activated Boc protected antimitotic agent and coupling said activated Boc protected antimitotic agent with a bifunctional linker, deprotecting the Boc group to form a coupled bifunctional linker- antimitotic agent and coupling the bifunctional linker- antimitotic agent to the B- subunit of Shiga toxin is yet a further aspect of the invention. In this method a reactive hydroxyl group is activated by phosgene and then coupled with the amine group on the bifunctional linker.

In these two methods the linker is synthesized starting from 2-[N-tert-butyloxycarbonyl)-N-methylamino]ethanol. Generally N-methylethanol was added to alumina then (Boc)₂O is added. The residue is diluted in ethyl acetate (EtOAc) and the product is obtained as oil. After 2-[N-(tert-butyloxycarbonyl)-N-methylamino]bromoethane is synthesized using THF, CBr₄ and PPh₃.2-[N-(tert-butyloxycarbonyl)-N-methylamino]acetylthio-ethyl is then synthesized using bromide in anhydrous DMF, and potassium thioacetate. The product is purified and is obtained as brown oil. 2-[N-(tert-butylloxtcarbonyl)-N-methylamino]1-ethyl-2-pyridyldisulfide is then synthesized using dithiopyridine in anhydrous MeOH and MeONa. The mixture is further purified over silica gel. 2-[N-Methylamino]1-ethyl-2-pyridyl disulfide dihydrodichloride is obtained by addition of methylene chloride and a phosgene solution followed by triethylamine. After evaporation the residue is purified over silica gel and carbamoyl chloride is obtained.

After synthesis of the linker the anti-mitotic agent, as described herein, and especially the monomethyl auristatin E is coupled to the linker. This coupling involves the protection of the amine group to couple the carbamoyl chloride to the secondary alcohol. For this synthesis a Boc protecting group is utilized to protect the amine group in the presence of triethylamine in methylene chloride. This coupling is done in the presence of two bases of 4-dimethylaminopyridine (DMAP) and di-isopropyethyl amine. After the Boc protection, the final product is deprotected using trifluoroacidic acid in methylene chloride.

To couple the antimitotic agent and linker to the B-subunit of Shiga toxin, a mixture of DMSO/water containing a phosphate buffer at pH7.5 is utilized and mixed with the B-subunit of Shiga toxin overnight at ambient temperature. The prodrug obtained is STxB-(anti-mitotic agent)₅ and more specifically STxB-(MMAE)₅ or STxB-(MMAF)₅ is obtained.

A number of embodiments and/or aspects of the invention have been described. Nevertheless it will be understood that various modifications may be made without departing from the spirit and scope of the invention.

### Examples

### Materials and methods

All reactions were performed under argon atmosphere unless otherwise noted.

### Solvents:

THF was distilled from sodium and benzophenone and CH₂Cl₂ was distilled from P₂O₅. DMA, DMF and mesitylene were stored over molecular sieves. Cyclohexane and toluene were distilled. Other solvents were used without further purification.

### Analyses:

Thin layer chromatography analyses were performed over Merck 60 F254 sheets and visualized with UV light.

Flash chromatographies were carried out on silica gel 320-400 mesh. Yields refer to chromatographically and spectroscopically pure materials.

IR spectra were recorded with a Fourier transform infrared spectroscopy (PERKIN-ELMER 1710 spectrometer).

Melting points were determined for recrystallised compounds only, by capillary method and are uncorrected.

Positive and negative electrospray ionization spectra were performed on a WATERS ZQ 2000.

High resolution mass spectra were obtained at the Laboratory of Mass Spectroscopy of ICSN CNRS(Laboratoire de Spectrométrie de Masse of ICSN CNRS) (Gif-sur-Yvette), or from Orleans.

¹H NMR and ¹³C NMR spectra were recorded at room temperature with a BRUCKER ACP 300 at respectively 300 MHz and 75 MHz with complete proton decoupling. Chemical shifts are reported in ppm relative to the residual solvent peak as the internal reference, coupling constants *j* are given in Hertz. Spin multiplicities are given with the following abbreviations: s = singlet, d = doublet, dd = doublet of doublet, t = triplet, q = quadruplet, m = multiplet. Peak assignment was unambiguously performed using DEPT, HMQC and HMBC techniques.

Optical rotation was measured when required using a Perkin-Elmer 241 polarimeter.

### Example 1-Synthesis of the linker

### A. Synthesis of 2-[N-(tert-butyloxycarbonyl)-N-methylamino]ethanol

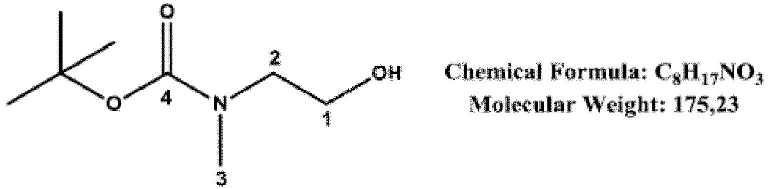

*N*-methylethanol (6.0 g, 0.08 mol) was added to alumina (12.4 g, 0.12 mol). Then 9.6 g (0.09 mol, 1.1 eq) of (Boc) 20 were added. The reaction was stirred for 10 min at room temperature. The residue was diluted in EtOAc (2x150 mL), filtered and evaporated. Mono protected compound (13.6 g, 97%) was obtained as an oil.
**Rf** : 0.25 (cyclohexane/EtOAc 8:2).
**1H NMR** (300 MHz, CDCl₃): 3.72 (t, 2H, *J* = 5,2 Hz, **H2**), 3.37 (d, 2H, *J* = 5,4 Hz, **H1**), 2.90 (s, 3H, **H3**), 2.59 (bs, 1H, O**H**), 1.44 (s, 9H, (**C**H₃)₃).
**13C NMR** (75 MHz, CDCl₃) : 157.1 (**C4**), 79,7 (**C**(CH₃)₃), 61.1 (**C2**), 51.2 **(C1),** 35.3 (**C3**), 28.0 ((**C**H₃)₃).
**MS (IC/NH3):** *m*/*z* = 176 [M+H]+.

### B. Synthesis of 2-[N-(tert-butyloxycarbonyl)-N-methylamino]ethanol

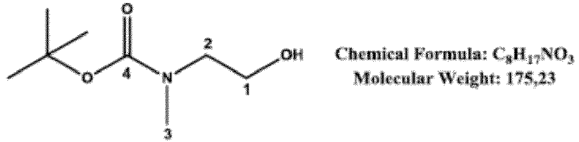

The process of EI Aloui A., et al., Angew. Chem. Int. Ed. 2006, 46, 6469-72 was followed. More specifically, *N*-methylethanol (6.0 g, 0.08 mol) was added to alumina (12.4 g, 0.12 mol). Then 9.6 g (0.09 mol, 1.1 eq) of (Boc)2O were added. The reaction was stirred for 10 min at room temperature. The residue was diluted in EtOAc (2x150 mL), filtered and evaporated. Mono protected compound (13.6 g, 97%) was obtained as an oil.
**Rf** : 0.25 (cyclohexane/EtOAc 8:2).
**1H NMR** (300 MHz, CDCl₃): 3.72 (t, 2H, *J* = 5,2 Hz, **H2**), 3.37 (d, 2H, *J* = 5,4 Hz, **H1**), 2.90
(s, 3H, **H3**), 2.59 (bs, 1H, O**H**), 1.44 (s, 9H, (**C**H₃)₃).
**13C NMR** (75 MHz, CDCl₃) : 157.1 (**C4**), 79,7 (**C**(CH₃)₃), 61.1 (**C2**), 51.2 (**C1**), 35.3 (**C3**), 28.0 ((**C**H₃)₃).
**MS (IC/NH3):** *m*/*z* = 176 [M+H]+.

### C. 2-[N-(tert-butyloxycarbonyl)-N-methylamino]bromoethane

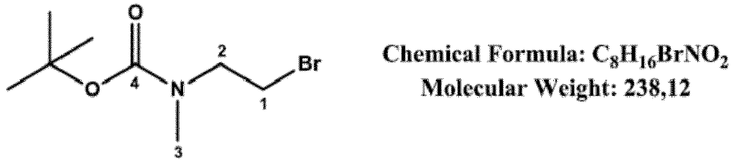

To a solution of 2-[*N*-(*tert*-butyloxycarbonyl)-*N-*methylamino]ethanol (220 mg, 1.25 mmol) in 5 mL of THF were added 622 mg (1.88 mmol, 1.5 eq) of CBr4 and 492 mg (1.88 mmol, 1.5 eq) of PPh3. The mixture was stirred at room temperature for 1 h. After filtration and washing with THF, the residue was evaporated and purified over silica gel (eluent: cyclohexane/EtOAc 80:20). Bromide **59** (59 mg, 20%) was obtained as an oil.
**Rf :** 0.4 (cyclohexane/EtOAc 80:20).
**1H NMR** (300 MHz, CDCl₃) : 3.57 (m, 2H, **H1**), 3.42 (m, 2H, **H₂**), 2.91 (s, 3H, **H3**), 1.45 (s, 9H, (C**H**₃)₃).
**13C NMR** (75MHz, CDCl₃) : 158.8 (**C4**), 79.9 (**C**(CH₃)₃), 50.7 (**C1**), 31.1 (**C3**), 29.2 (**C2**), 28.3 ((**C**H₃)₃).
**MS** (IC/NH3) : *m*/*z* = 238 [M+H]+.

### D.Synthesis of 2-[N-(tert-butyloxycarbonyl)-N-methylamino]acetylthio-ethyl

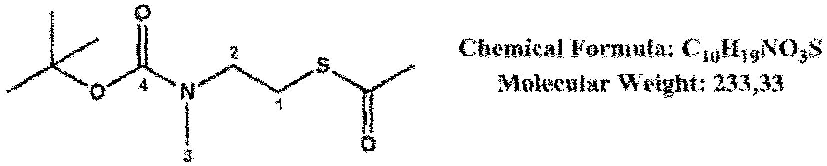

### Method A:

To a solution of 2-[*N*-(*tert*-butyloxycarbonyl)-*N-*methylamino]bromoethane (350 mg, 1.46 mmol) in 8 mL of anhydrous DMF, potassium thioacetate (184 mg, 1.61 mmol, 1.1 eq) was added under argon. The mixture was stirred at room temperature during 1 h. After evaporation under vacuum and purification (eluent: cyclohexane/EtOAc 80:20), thioacetate, 2-[*N*-(*tert*-butyloxycarbonyl)-*N*-methylamino]acetylthioethyl (270 mg, 79%) was obtained as a brown oil.

### Method B:

To a solution of *N*-methylethanol (3.5 g, 20 mmol) in 100 ml of THF at 0°C was added thiolacetic acid (2.0 ml, 26.73 mmol) and triphenylphosphine (7.0 g, 26.73 mmol) under argon. After stirring at 0°C for 15 min, DIAD (6.0 ml, 28.93mmol) was added. The mixture was stirred at 0°C for 2 h then 5 h at room temperature (RT). The mixture was concentrated, diluted with 120 ml of EtOAc/Hexane (1:2), filtered through celite. The solution was washed with saturated NaHCO₃ and brine respectively, dried over MgSO₄, filtered, evaporated and purified on SiO₂ chromatography eluted with EtOAc/hexane (90:10 to 80:20) to afford (4.0 g, 86%) of 2-[*N*-(*tert*-butyloxycarbonyl)-*N*-methylamino]acetylthio-ethyl.
**Rf:** 0.3 (CH₂Cl₂/acetone: 80:20).
**1H NMR** (300 MHz, CDCl₃): 3.34 (t, 2H, *J* = 6,8 Hz, **H1**), 3.00 (t, 2H, *J* = 6,8 Hz, **H2**), 2.90.
(s, 3H, **H3**), 2.33 (s, 3H, COC**H**₃), 1.45 (s, 9H, (C**H**₃)₃).
**13C NMR** (75 MHz, CDCl3) : 195.2 (S**C**O), 155.4 (**C4**), 79.5 (**C**(CH₃)₃), 48.2 (**C2**), 34.4 (**C3**), 30.5 (**C1**), 28.4 ((**C**H3)3), 27.1 (SCO**C**H3).
**MS (IC/NH₃):** *m*/*z* = 234 [M+H]+.

### E. Synthesis of 2-[N-(tert-butyloxycarbonyl)-N-methylamino]1-ethyl 2-pyridyldisulfide

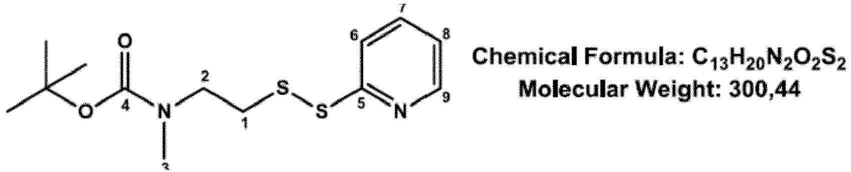

To a solution of thioacetate (2-[*N*-(*tert*-butyloxycarbonyl)-*N-*methylamino]acetylthio-ethyl) (3 g, 12.8 mmol) and dithiopyridine (2.8 g, 0.643 mmol) in 90 mL of anhydrous MeOH, were added at 0 °C, 4 mL of MeONa 1M in MeOH. The mixture was stirred during 30 min at room temperature, and quenched with 30 mL of water, extracted with EtOAc (2 x 100 mL), washed with brine, dried over MgSO₄ and evaporated. The residue was chromatographied over silica gel (eluent: CH₂Cl₂/acetone: 98/2) in order to isolate 2.7 g (70%) of 2-[*N*-(*tert*-butyloxycarbonyl)-*N-*methylamino]1-ethyl 2-pyridyldisulfide as a brown oil.
**Rf:** 0.54 (CH₂Cl₂/acetone: 98:2).
**1H NMR (300 MHz, CDCl₃) :** 8.48 (d, 1H, *J* = 4,8 Hz, **H9**), 7.67-7.60 (m, 2H, **H6, H7**), 7.10 (dd, 1H, *J* = 6,6 Hz et j' = 4,8 Hz, **H8**), 3.61 (t, 2H, *J* = 6,9 Hz, **H1),** 2.92 (t, 2H, *J* = 4,2 Hz, **H2**), 2.87 (s, 3H, **H3**), 1.45 (s, 9H, (C**H**₃)₃).
**13C NMR (CDCl₃) :** 159.7 (**C5**), 155.3 (**C4**), 149.6 (**C9**), 137.3 (**C7**), 121.1 et 120.7 (**C8, C6**), 79.7 (**C**(CH₃)₃), 48.1 (**C1**), 36.2 (**C3**), 34.5 (**C2**), 28.3 ((**C**H₃)₃).
**MS (IC/NH3):** *m*/*z* = 301 [M+H]+

### F. Synthesis of 2-[N-Methylamino]1-ethyl 2-pyridyl disulfide dihydrodichloride

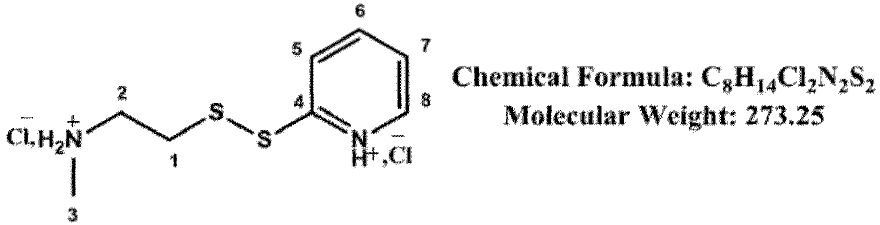

A. Disulfide (2.5 g, 8.3 mmol) was suspended in 150 mL 1.5 M HCl in EtOAc. After 2 h at room temperature, no more starting material could be seen by TLC. The mixture was then evaporated to dryness and the quantitatively obtained chlorhydrate (2-[*N*-Methylamino]1-ethyl 2-pyridyl disulfide dihydrodichloride was directly used in the next step.
**Rf:** 0.00 (CH₂Cl₂/acetone : 95:5).
**1H NMR (300 MHz, CDCl3) :** 8.71 (d, 1H, *J* = 4,7 Hz, **H8**), 8.33-7.97 (m, 2H, **H5, H6**), 7.68 (dd, 1H, *J* = 6,5 Hz and *J*' = 4,7 Hz, **H7**), 3.38 (m, 2H, **H1**), 3.23 (t, 2H, *J* = 6.9 Hz, **H2**), 2.77 (s, 3H, NC**H**₃).
**MS (IC/NH3):** *m*/*z* = 201 [M+H-2HCl]+.

### G. Synthesis of 2-[N-(Chlorocarbonyl)-N-methylamino]1-ethyl 2-pyridyldisulfide

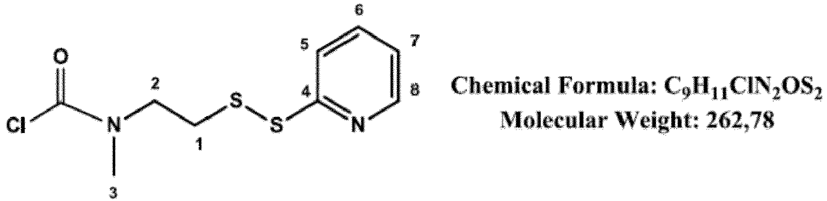

To a suspension of 2.2 g (8.0 mmol) of chlorhydrate (2-[*N-*Methylamino]1-ethyl 2-pyridyl disulfide dihydrodichloride) in 150 mL of freshly distilled CH₂Cl₂, 22 mL (excess) of a 20% phosgene solution in toluene were added at 0 °C, followed by Et3N. (2.2 mL,16mmol). The icebath was removed and the stirring pursued for 2.5 h at room temperature. After evaporation, the residue was purified over silica gel (eluent: CH₂Cl₂/acetone: 95/5). 2-[*N*-(Chlorocarbonyl)-*N*-methylamino]1-ethyl 2-pyridyldisulfide (1.6 g, 78%) was obtained as a colorless oil.

### Example 2-Synthesis of tert-butyl ((S)-1-(((S)-1-(((3R,4S,5S)-1-((S)-2-((1R,2R)-3-(((1R,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate

A solution of di-*tert*-butyl dicarbonate (1.2 mg, 0.058 mmol) in CH₂Cl₂ (1 ml) was added dropwise to a stirred solution of MMAE (41.8 mg, 0.058 mmol) and Et₃N (8 µL, 0.058 mmol) in CH₂Cl₂ (1 ml).

The reaction mixture was stirred for 2 h and was then washed with saturated aqueous sodium hydrogen carbonate solution, water and then brine.

The organic layer was dried over MgSO₄ and the volatiles were removed by evaporation to give the title product set forth below (45.1 mg, 95 %) as a colorless oil. 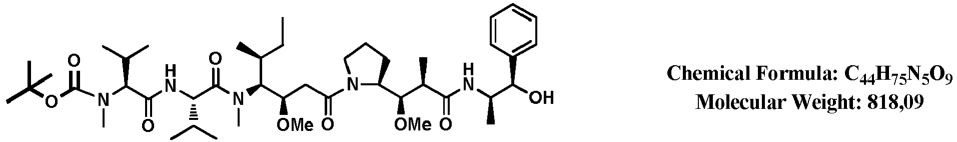
**Rf:** 0.35 (CH₂Cl₂/MeOH : 96:4)
**¹H NMR** (CDCl₃, 300 MHz) δ 7.35-7.26 (m, 5H, **Ph**), 6.41-6.51 (br, 1H), 5.61 (d, *J* = 4.9 Hz, 1H), 4.71-4.62 (br, 1H), 4.49-4.38 (m, 1H), 4.19-4.03 (m, 2H), 3.82 (d, *J* = 4.8 Hz, 1H), 3.75-2.81 (m, 9H), 2.51-0.84 (m, 54H) **MS** (ESI): *m*/*z* 840 [M+Na]⁺
**MALDI TOF MS :** *m*/*z* 818 [M+H]⁺

### Example 3-Synthesis of (1R,2R)-2-((2R,3R)-3-((S)-1-((6S,9S,12S,13R)-12-((S)-sec-butyl)-6,9-diisopropyl-13-methoxy-2,2,5,11-tetramethyl-4,7,10-trioxo-3-oxa-5,8,11-triazapentadecan-15-oyl)pyrrolidin-2-yl)-3-methoxy-2-methylpropanamido)-1-phenylpropyl methyl(2-(pyridin-2-yldisulfanyl)ethyl)carbamate

**Step 1.** 20 percent phosgene in toluene solution ( 2 ml 0.4 mmol) was cooled to -10°C under argon and Boc-auristatin E (40 mg, 0.049 mmol) in dichloromethane (0.5 ml) was added, followed by dropwise addition of triethylamine(6.9 µl 0.05 mmol) in dichloromethane (0.5 ml). The mixture was stirred for 1 hour. Nitrogen was introduced into the reactor through the reactor inlet tube to remove the excess phosgene. The reaction mixture was used in the next procedure without purification.

**Step 2.** The solution of activated Boc-auristatin was then added to a solution of 2-[N-methy;amino]1-ethyl 2-pyridyl disulfide dihydrochloride (16.4 mg. 0.06 mmol) and DIPEA ( 34.8 µl, 0.2 mmol) in dichloromethane (1 ml) at -10°C. The reaction was stirred at room temperature for 6 hours then diluted with dichloromethane. The solution was washed with 0.5 M hydrochloric acid, 5 percent sodium bicarbonate and water, dried (MgSO₄) and evaporated. The residue was purified by flash chromatography, and eluted with 2-4 percent MeOH/dichloromethane to give the following compound as a yellow solid (46 mg, 90%). 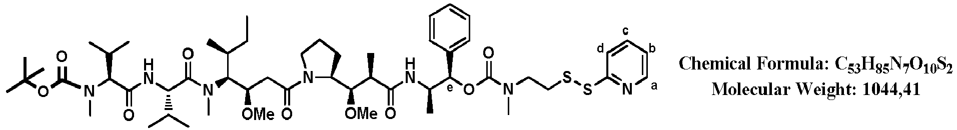
**Mp:** 146 °C
**Rf:** 0.35 (CH₂Cl₂/MeOH : 96:4)
**¹H NMR** (CDCl₃, 300 MHz) δ 8.45 (d, *J* = 4.5 Hz, 1H, **Ha**), 7.67-7.63 (m, 2H, **Hb, Hc),** 7.35-7.26 (m, 5H, **Ph**), 7.10-7.08 (m, 1H, **Hd**), 6.41-6.51 (br, 1H), 5.61 (d, *J* = 4.9 Hz, 1H, **He**), 4.71-4.62 (br, 1H), 4.49-4.38 (m, 1H), 4.19-4.03 (m, 2H), 3.82 (d, *J* = 4.8 Hz, 1H), 3.75-2.81 (m, 15H), 2.51-0.84 (m, 54H)
**MS** (ESI): *m*/*z* 1066 [M+Na]⁺
**MALDI TOF MS**: *m*/*z* 1044 [M+H]⁺

### Example 4-Synthesis of (1R,2R)-2-((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropanamido)-1-phenylpropyl methyl(2-(pyridin-2-yldisulfanyl)ethyl)carbamate

To a solution of (1R,2R)-2-((2R,3R)-3-((S)-1-((6S,9S,12S,13R)-12-((S)-sec-butyl)-6,9-diisopropyl-13-methoxy-2,2,5,11-tetramethyl-4,7,10-trioxo-3-oxa-5,8,11-triazapentadecan-15-oyl)pyrrol id in-2-yl)-3-methoxy-2-methylpropanamido)-1-phenylpropyl methyl(2-(pyridin-2-yldisulfanyl)ethyl)carbamate (27 mg, 0.026 mmol) in 0.5 mL dry CH₂Cl₂ was added 0.5 mL of TFA. The yellow solution was stirred at room temperature over 2 h. After completion of the reaction, the mixture was reduced under vacuum and the residue was purified by chromatography (7 % MeOH in CH₂Cl₂), affording 22.8 mg (93 % yield) of the compound below as a deep white solid. 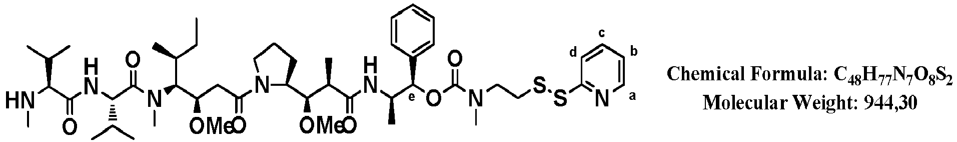
**Rf:** 0.4 (CH₂Cl₂/MeOH, 90:10)
**¹H NMR** (CD₃OD, 300 MHz) δ 8.42 (d, *J* = 4.5 Hz, 1H, **Ha**), 7.68-7.64 (m, 2H, **Hb, Hc**), 7.36-7.25 (m, 5H, **Ph**), 7-09-7.08 (m, 1H, **Hd**), 6.41-6.51 (br, 1H), 5.61 (d, *J* = 4.9 Hz, 1H, **He**), 4.71-4.62 (br, 1H), 4.49-4.38 (m, 1H), 4.19-4.03 (m, 2H), 3.82 (d, *J* = 4.8 Hz, 1H), 3.75-2.81 (m, 15H), 2.51-0.84 (m, 45H).
**MS** (ESI): *m*/*z* 967 [M+Na]⁺
**MALDI TOF MS:** *m*/*z* 945 [M+H]⁺

### Example 5-Ligation of STxB to (1R,2R)-2-((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropanamido)-1-phenylpropyl methyl(2-(pyridin-2-yldisulfanyl)ethyl)carbamate

A mixture STxB (2.5 mg/mL, 0.3 µmol) in 20 mM phosphate buffer pH 7.5 was incubated volume to volume with a 1, 3, 9 fold molar excess of compound (1R,2R)-2-((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropanamido)-1-phenylpropyl methyl(2-(pyridin-2-yldisulfanyl)ethyl)carbamate dissolved in DMSO. The residue was purified by gel filtration over PD 10 column and dialysed to afford quantitatively the desired conjugate set forth below:

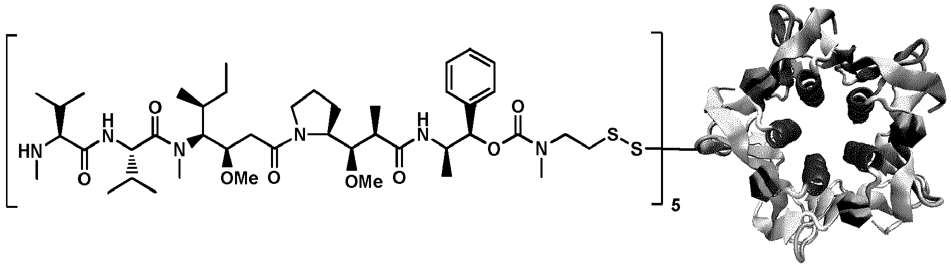

**MALDI TOF MS:** Compound 78 set forth above *m*/*z* 8623 calculated, 8623 found
(STxB-SH *m*/*z* 7793 calculated, 7790 found).

### Example 6-Retrograde transport of STxB-MMAE (auristatin) cleavable conjugate

24h before contact, HeLa cells were seeded on glass slides at a final density of 70,000 cells/well, in DMEM supplemented with 10% FCS. Binding of STxB-MMAE cleavable conjugates to HeLa cells was performed at 4°C for 30 min at a final concentration of 0.2 µM (STxB monomer); cells were then washed twice in complete medium and incubated for 40 min at 37°C. The intracellular localization of STxB-MMAE conjugates was characterized by immunofluorescence detection after cell fixation in 4% PFA. Retrograde transport of the conjugate was confirmed by co-localization of STxB with Golgi markers, observed by epifluorescence microscopy.

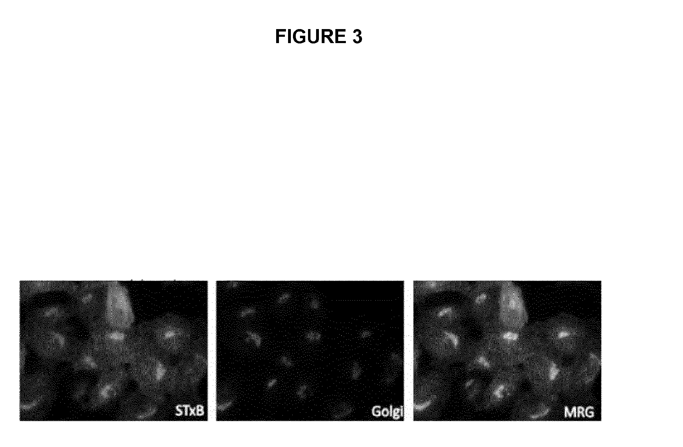

The superposition at the far right of Figure 3 of the images of STxB and in the Golgi validated the retrograde transport of this conjugate within the cells despite the presence of the cyctotoxic agent (MMAE)₅

### Example 7-In vitro cytotoxicity of STxB-MMAE (auristatin) cleavable conjugate

24 hours before contact, HeLa cells were seeded in 96 well plates at a final density of 3000 cells/well, in DMEM supplemented with 10% FCS. For demonstration of Gb3-dependent toxicity of conjugates, HeLa cells were also treated during 5 days with PPMP (1-phenyl-2-palmitoylamino-3-morpholino-1-propanol (5uM) to inhibit glycosphingolipid synthesis.

Cells treated or not with PPMP, were incubated with increasing concentrations (up to 1 µM) of STxB-MMAE cleavable conjugate, pro-MMAE (chemically modified MMAE for coupling to STxB) or MMAE, for 6 hours in DMEM complete medium at 37°C. Cells were then washed and incubated for 5 days at 37°C, 5% CO₂. Cell viability was determined by detection of mitochondrial metabolism using the colorimetric tetrazolium salt-based (MTT) assay. Results were normalized to non-treated cells; each point was determined in triplicate.

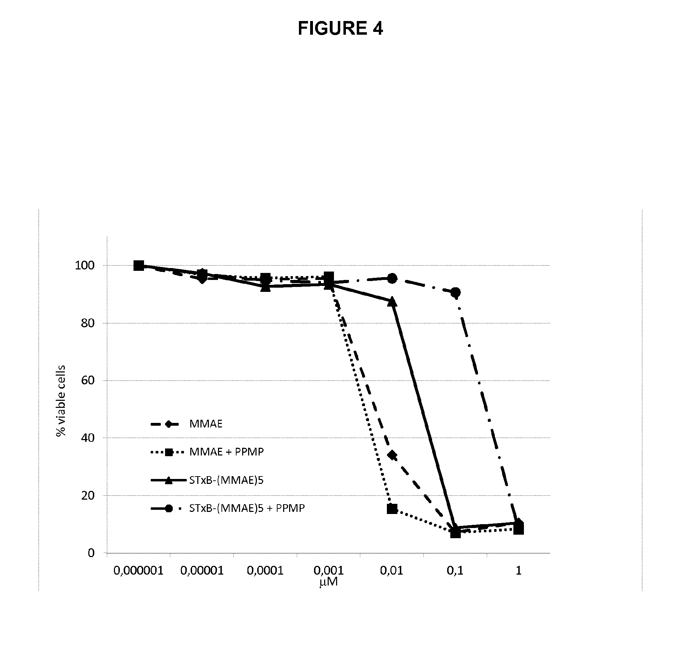

The results in Figure 4 show that the cytotoxity of the STxB-(MMAE)₅ conjugate, having an IC₅₀ of 50 nM was six times better as compared to the synthetic conjugate STxB-(SN38)₅, which had an IC₅₀ of 300 nM.

### Example 8-In Vivo Activity of STxB-(MMAE)₅

Ten APV^{1638N} 6 month old mice are injected 3 times intravenously at days 1, 8 and 15 using the STxB-(MMAE)₅ conjugate. As a control only STxB is injected at the same molar dose. At day 28 after the first injection the mice are sacrificed and their intestines are analyzed macroscopically on autopsy preparation for the presence of periampular tumors. The same preparations are then treated for pathological examinations. The presence of periampular tumours in treated and untreated mice are determined by macroscopical observation and pathological analysis.

This experiment clearly establishes that STxB-(MMAE)₅ conjugate protects the animal from tumor growth and also induces tumor regression.

A number of embodiments and/or aspects of the invention have been described. Nevertheless it will be understood that various modifications may be made without departing from the spirit and scope of the invention.

## Claims

1. A conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group.

2. The conjugate according to Claim 1, wherein said antimitotic agent has a reactive hydroxyl, amino or thiol group and is selected from the group comprising halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins and derivatives, doxorubicin and derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, platinum derivatives, auristatin, auristatin E, monomethyl auristatin E, pharmaceutically acceptable salts thereof and mixtures thereof.

3. The conjugate according to Claim 1 or 2 wherein the functional equivalent of the Shiga B toxin is selected from the group of Shiga-like toxin 1, Shiga-like toxin 2 , Shiga-like toxin 2c, Shiga-like toxin 2d1, Shiga-like toxin 2d2, Shiga-like toxin 2e, Shiga-like toxin 2f and Shiga-like toxin 2y,verotoxin-1, verotoxin-2, verotoxin 2c or verotoxin 2v.

4. The conjugate according to any one of Claims 1 to 3, wherein the carbamate group couples the at least one antimitotic agent and the disulfide group couples the at least one B-subunit of Shiga toxin or functional equivalents thereof through the free thiol group.

5. The conjugate according to any one of claims 1 to 4, wherein said carbamate group has the formula NCOO and the disulfide group is a di-thiol pyridine group having the formula S₂-C₅H₅N.

6. A pharmaceutical composition comprising the conjugate according to any one of Claims 1 to 5 and a pharmaceutically acceptable vehicle.

7. The pharmaceutical composition according to Claim 6, comprising an additional therapeutic agent.

8. The pharmaceutical composition according to Claim 7, wherein the additional therapeutic agent is selected from the group of an analgesic, an antimicrobial, an antibacterial, an antiviral, an antibiotic, an anti-inflammatory, an antioxidant, an antihistamine, an antipruritic, an antipyretic, an additional anti-cancer agent and combinations thereof.

9. The pharmaceutical composition according to any one of Claims 6 to 8, wherein 5 antimitotic agents are coupled to the B-subunit of Shiga toxin.

10. The conjugate according to any one of Claims 1 to 5 or the pharmaceutical composition according to any one of Claims 6 to 9 for use as a medicament.

11. The conjugate or pharmaceutical composition according to Claim 10, for use in treating cancer.

12. The use according to Claim 11, wherein said cancer is selected from the group comprising acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt Lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ*, embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ*, lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vaginal cancer and vulvar cancer.

13. A pharmaceutical composition comprising a first conjugate comprising at least one B-subunit of Shiga toxin or a functional equivalent thereof covalently coupled to at least one antimitotic agent or pharmaceutically acceptable salts thereof through a linker, wherein said linker comprises a bifunctional linker connecting the at least one antimitotic agent or pharmaceutically acceptable salt thereof through a carbamate group and the at least one B-subunit of Shiga toxin or functional equivalent thereof through a disulfide group and a second conjugate comprising at least one B-subunit of Shiga toxin or a functional equivalent thereof covalently coupled to at least one cancer antigen through a linker, wherein said linker comprises a bifunctional linker connecting the at least one cancer antigen through a carbamate group and the at least one B-subunit of Shiga toxin or functional equivalent thereof through a disulfide group and a pharmaceutically acceptable vehicle.

14. A method of making a conjugate according to any one of Claims 1 to 5, said method comprising synthesizing a bifunctional linker, reacting a Boc protected antimitotic agent with said bifunctional linker, deprotecting the Boc group on said antimitotic agent to form a coupled bifunctional linker- antimitotic agent and coupling the bifunctional linker- antimitotic agent to the B-subunit of Shiga toxin.

15. A method of making a conjugate according to any one of Claims 1 to 5, said method comprising reacting a hydroxyl group of a Boc protected antimitotic agent to form an activated Boc protected antimitotic agent and coupling said activated Boc protected antimitotic agent with a bifunctional linker, deprotecting the Boc group to form a coupled bifunctional linker-antimitotic agent and coupling the bifunctional linker- antimitotic agent to the B-subunit of Shiga toxin.
